# EUROPÄISCHE PATENTSCHRIFT

(11) **EP 0 582 582 B1**
(45) Veröffentlichungstag und Bekanntmachung des Hinweises auf die Patenterteilung: **17.09.1997**
(21) Anmeldenummer: 92907094.4
(22) Anmeldetag: 27.03.1992
(51) Int. Cl.: C12Q 1/68, G01N 33/574, C07K 2/00, C07K 4/00, C07K 14/00, C07K 16/00

(54) **VERFAHREN ZUM NACHWEIS DER DIFFERENZIERUNG UND DER INVASIVITÄT VON KARZINOMZELLEN**
PROCESS FOR DETECTING THE DIFFERENTIATION AND INVASIVENESS OF CARCINOMA CELLS
PROCEDE DE DETECTION DE LA DIFFERENTIATION ET DE L'INVASIVITE DE CELLULES DE CARCINOMES

(30) Priorität: 28.03.1991 DE 4110405
(43) Veröffentlichungstag der Anmeldung: 16.02.1994
(73) Patentinhaber: MAX-DELBRÜCK-CENTRUM FÜR MOLEKULARE MEDIZIN, D-13125 Berlin (DE)
(72) Erfinder: BIRCHMEIER, Walter, D-4300 Essen 1 (DE); FRIXEN, Uwe, D-4300 Essen 1 (DE); BEHRENS, Jürgen, D-4300 Essen 1 (DE)
(74) Vertreter: Baumbach, Friedrich
(86) Internationale Anmeldenummer: EP9200692
(87) Internationale Veröffentlichungsnummer: WO9217608

(56) Entgegenhaltungen:
- JOURNAL OF CANCER RESEARCH AND CLINICAL ONCOLOGY, Bd. 116/S, 1990, BERLIN, GERMANY, Seite 182; J.PFISTERER ET AL.: 'ARC-1/uvomorulin by immunohistochemistry in non malignant tissues and in ovarian tumors'
- DIFFERENTIATION, Bd. 38, Nr. 1, Juni 1988, BERLIN, GERMANY, Seiten 67-71; A. MANSOURI et al.: "Characterization and chromosomal localization of the gene encoding the human cell adhesion molecule uvomorulin"
- JOURNAL OF CELL BIOLOGY, Bd. 108, Nr. 6, Juni 1989, NEW YORK, US, Seiten 2435-2447; J.BEHRENS ET AL.: 'Dissecting tumor cell invasion: Epithelial cells acquire invasive properties after the loss of uvomorulin-mediated cell-cell adhesion'
- JOURNAL OF CELL BIOLOGY, Bd. 113, Nr. 1, April 1991, NEW YORK, US, Seiten 173-185; U.H.FRIXEN ET AL.: 'E-cadherin -mediated cell-cell adhesion prevents invasiveness of human carcinoma cells'

## Beschreibung

Die Erfindung betrifft ein Verfahren zum Nachweis der Differenzierung und Invasivität von Tumorzellen und Tumoren epithelialen Ursprungs.

Die Karzinogenese ist gewöhnlich ein mit vielen Schritten ablaufender Prozeß, der unter anderem die Aktivierung, Mutation oder den Verlust von verschiedenen Genen einschließt. In den betroffenen Zellen bewirken diese Veränderungen den Verlust der Wachstumskontrolle, die Invasion von benachbartem Gewebe, Attraktion von Blutgefäßen (Angiogenese) oder die Metastasenbildung auch in entfernten Organen (siehe hierzu auch Nicholson, Biochim. Biophys. Acta 695 (1982), 113-176; Klein und Klein, Nature 315 (1985), 190-1995). Für einige dieser Tumor-Progressionsschritte ist die Veränderung von zellulären Wechselwirkungen notwendig und daher spielen Zelladhäsionsmoleküle dabei eine wichtige Rolle.

Karzinome können aufgrund von morphologischen Kriterien unterteilt werden. Es gibt differenzierte ("well differentiated") Karzinome, die im wesentlichen die ursprünglichen epithelialen Zellstrukturen beibehalten und z.B. gut entwickelte interzelluläre Verbindungen besitzen. Diese Art von Karzinomen sind meist schwach oder gar nicht invasiv. Im Gegensatz dazu gibt es entdifferenzierte ("poorly differentiated") Karzinome, die eine mehr amorphe Gewebestruktur besitzen und weniger interzelluläre Verbindungen aufweisen. Diese Art von Karzinomen ist oft stärker invasiv (Gabbert et al., Clin. Exp. Metastasis 3 (1985) 257-279). Da nachgewiesen wurde, daß das Ausmaß an Differenzierung und die Stärke der Invasivität verschiedener Karzinome oft die Überlebensprognose von Patienten bestimmt (Morson und Dawson (1979), Gastrointestinal Pathology, 2nd ed., Blackwell), besteht großes Interesse an der Aufklärung der molekularen Charakteristika der einzelnen Karzinomklassen.

In den vergangenen Jahren wurde eine Reihe von spezifischen Zell-Zell- und Zell-Substrat-Adhäsionsmolekülen identifiziert und untersucht. Dabei wurde unter anderem festgestellt, daß das Zell-Zell-Adhäsionsmolekül, welches mit E-Cadherin (Arc-1, Uvomorulin, L-CAM oder cell-CAM 120/80) bezeichnet wird, eng mit der Entstehung von invasiven Karzinomen verbunden ist. E-Cadherin ist ein 120 kd großes Zelloberflächenglykoprotein, von dem in Gegenwart von Ca²⁺ ein lösliches 80 kd großes Trypsin-Fragment abgespalten werden kann, und das als 135 kd Vorläuferprotein synthetisiert wird (siehe z.B. Peyrieras et al., Proc. Natl. Acad. Sci. USA 80 (1983), 6274-6277). Es wurde festgestellt, daß nicht transformierte Epithelzellen dieses Protein auf ihrer Oberfläsche tragen, die Blockierung dieses Proteins durch einen monoklonalen Antikörper aber dazu führt, daß die Zellen invasiv werden, und schließlich, daß viele transformierte Epithelzellen dieses Protein nicht auf ihrer Oberfläche tragen (Behrens et al., J. Cell Biol. 108, 2435-2447 (1989).

Das Dokument Journal of Cancer Research and Clinical Oncology 116/s, 182 (1990) offenbart, daß die Intensität einer immunhistochemischen E-Cadherin-Anfärbung in unterschiedlichen Eierstock-Tumoren eine Korrelation mit der Tumordifferenzierung zeigt. Eine generelle Korrelation zwischen E-Cadherin und der Invasivität von Tumoren epithelischen Ursprungs wird nicht beschrieben.

Wie man weiß, sind 90 % der malignen menschlichen Tumoren Karzinome, welche aus transformierten Epithelzellen entstehen und das darunterliegende Mesenchym invadieren können. Es wurde weiter festgestellt, daß die Invasivität mit der Bösartigkeit der Tumoren verbunden ist, wogegegen gutartige Tumoren nicht invasiv sind. Diese können zwar stark expansiv sein und eine enorme Größe erreichen, jedoch invadieren sie Bindegewebe nicht. Bösartige Tumoren sind dagegen oftmals klein, jedoch invadieren sie die mesenchymalen Strukturen leicht und bilden dadurch schnell Metastasen im ganzen Körper.

Es sind bereits einige Verfahren bekannt, welche die Klassifizierung von bestimmten Karzinomen erlauben. So kann durch Untersuchung von Keratinen und intermediären Filamentproteinen eine Unterscheidung von Tumoren aus verschiedenen Geweben erfolgen (Osborn und Weber, Lab. Invest. 48 (1983), 372-394).

Weiterhin können desmosomale Proteine als Marker zur Identifizierung und Klassifizierung gewisser epithelialer Tumore dienen (Moll et al., Lab. Invest. 54 (1986), 4-25). Weiterhin kann für Karzinome des Verdauungstrakts das karzino-embryonische Antigen (CEA) als wertvoller Indikator dienen (Mentges, Dtsch. Med. Wschr. 112 (1987), 1245-1249). Der Östrogenrezeptor ist ebenfalls ein nützlicher Marker für das Differenzierungsstadium von Brustkarzinomen (Engel und Young, Cancer Res. 38 (1978), 4327-4339). Schließlich können auch Onkogene, z.B. das Ki-ras-Gen (Almoguera et al., Cell 53 (1988), 549-554) und Tumorsuppressorgene, z.B. das Retinoblastom-Suszeptibilitätsgen (Bookstein et al. (1990), Science 247, 712-715), das Wilms-Tumorgen (Haber et al. (1990), Cell 61, 1257-1269) oder das p53-Gen (Tsai et al. (1990), Cancer Res. 50, 44-47) als Indikatoren für bestimmte Karzinome dienen. Das Charakteristische aller dieser Verfahren ist jedoch, daß die dabei verwendeten Indikatoren jeweils nur bei ganz bestimmten Tumorarten verwendet werden können.

Es ist daher die Aufgabe der vorliegenden Erfindung, ein einfaches Verfahren zu entwickeln, welches eine generellere Bestimmung der Invasivität von epithelialen Tumorzellen erlaubt und es dadurch ermöglicht, eine schnelle und zuverlässige Unterscheidung zwischen gutartigen und bösartigen Tumorgeweben zu treffen.

Gelöst wird die erfindungsgemäße Aufgabe durch ein Verfahren zum Nachweis der Differenzierung oder/und Invasivität von Tumorzellen und Tumoren epithelialen Ursprungs, bei dem man die Expression des Oberflächenantigens E-Cadherin in Tumorzellen auf Proteinebene oder/und auf mRNA-Ebene untersucht. Dabei wurde gefunden, daß die Expression von E-Cadherin bei allen bisher untersuchten Tumorzellinien und Tumoren epithelialen Ursprungs proportional mit zunehmender Entdifferenzierung und Invasivität abnimmt.

Die verschiedenen getesteten differenzierten und entdifferenzierten menschlichen Karzinoma-Zellinien epithelialen Ursprungs sind von der Tumorbank des Deutschen Krebsforschungszentrums Heidelberg (DKFZ) und der American Type Culture Collection (ATCC) erhältlich. Es wurden folgende Zellinien verwendet: RT4 und RT112 (von differenzierten Blasen-Karzinomen), EJ28 (von einem entdifferenzierten Blasen-Karzinom), CX-1 WiDr, HCT116, SW948 und C0L0205 (von differenzierten Colon-Karzinomen), SW620 (von einem entdifferenzierten Colon-Karzinom), MCF-7 und MDA-MB-361 (von einem differenzierten Brust-Karzinom), BT-549 (von einem Brustkarzinom mit intermediärem Phänotyp), MDA-MB-231, -435S und -436 (von entdifferenzierten Brust-Karzinomen), LX-1 (von einem differenzierten Lungen-Karzinom), A-427 und A-549 (von Lungen-Karzinomen mit intermediärem Phenotyp), LXF 289 und SK-MES-1 (von entdifferenzierten Lungen-Karzinomen), Capan-1, Capan-2 und DAN-G (von differenzierten Pankreas-Karzinomen), Hs 766T (von einem Pankreas-Karzinom mit intermediärem Phenotyp) und MIA PaCa-2 (von einem entdifferenzierten Pankreaskarzinom). Detailliertere Hinweise auf diese Zellinien finden sich in Tabelle 1.

Bei Experimenten mit diesen Zellen wurde gefunden, daß bei allen untersuchten Tumor-Zellinien die E-Cadherin-Expression mit dem Differenzierungsstadium bzw. der Invasivität der jeweiligen Zellinie korreliert. Es ist daher anzunehmen, daß diese Korrelation nicht auf die oben erwähnten Zellinien beschränkt ist, sondern generell bei Tumorzellinien epithelialen Ursprungs gefunden wird.

Weiterhin wurden auch Untersuchungen an verschiedenen menschlichen Tumoren auf die Expression von E-Cadherin durchgeführt. Bei Larynx- und Pharynx-Plattenepithelkarzinomen, bei lobulären Brustkarzinomen sowie bei Ovarialkarzinomen wurde festgestellt, daß Normalgewebe und differenzierte Tumore E-Cadherin exprimieren, während weniger differenzierte ("moderately well differentiated") und dedifferenzierte Tumore wenig oder kein E-Cadherin exprimieren.

Zum Nachweis von E-Cadherin in Tumorzellen und Tumoren können prinzipiell zwei Verfahren verwendet werden. Die Expression von E-Cadherin kann auf Proteinebene oder/und auf mRNA-Ebene untersucht werden.

Ein Gegenstand der Erfindung ist auch die Verwendung von monoklonalen Antikörpern gegen E-Cadherin zum Nachweis der Differenzierung und Invasivität von Tumorzellen, wobei man die Tumorzelloberfläche oder Gewebeschnitte auf Anwesenheit von E-Cadherin durch Bindung eines der erfindungsgemäßen monoklonalen Antikörper in markierter Form untersucht. Hierbei kann davon ausgegangen werden, daß Zellen oder Gewebe, welche E-Cadherin an ihrer Oberfläche tragen, weniger invasiv sind und daher die Gefahr der Invasion und Metastasierung der Zellen geringer ist. Weisen die Zelloberflächen oder Gewebeschnitte jedoch kein E-Cadherin auf, an das die monoklonalen Antikörper binden, muß davon ausgegangen werden, daß es sich um eher bösartige, nämlich invasive Tumorzellen handelt.

Für die Durchführung des erfindungsgemäßen Verfahrens zum Nachweis der Invasivität oder/und Differenzierung von Tumorzellen mit Hilfe von markierten monoklonalen Antikörpern sind alle an sich bekannten Arten der Markierung des Antikörpers, sowie des Nachweises der Markierung und alle für die Untersuchung mit einem monoklonalen Antikörper gebräuchlichen Testanordnungen geeignet. Zur Markierung der erfindungsgemäßen monoklonalen Antikörper werden vorzugsweise Fluoreszenzmarkierungen eingesetzt, wodurch eine einfache Detektion ermöglicht wird. Weiter kann jedoch auch beispielsweise eine Immuno-Gold-Färbung durchgeführt werden.

In einer bevorzugten Ausführungsform des erfindungsgemäßen Verfahrens werden Extrakte von Tumorzellen (d.h. Gesamtextrakte oder proteolytisch behandelte Extrakte) auf die Anwesenheit von E-Cadherin oder eines Fragments davon durch Bindung von Antikörpern gegen E-Cadherin untersucht. Besonders bevorzugt für dieses Verfahren ist die Verwendung des monoklonalen Antikörpers 6F9 zum Nachweis von E-Cadherin oder eines Fragments davon.

Die erfindungsgemäßen monoklonalen Antikörper erkennen und binden spezifisch an das Zell-Adhäsionsmolekül E-Cadherin. Der bevorzugte Antikörper 6F9 wird von den bei der GBF Braunschweig hinterlegten Hybridomazellinie 6F9 (Hinterlegungsnummer ACC 1006) sekretiert.

Zum Nachweis von E-Cadherin sind jedoch neben den genannten Antikörpern ebenso auch andere monoklonale Antikörper (z.B. 15G7 und 15C12) gegen dieses Protein geeignet. Die Herstellung solcher Antikörper kann auf übliche Weise durch Immunisierung von Versuchstieren mit E-Cadherin oder einem Fragment davon und anschließender Gewinnung der resultierenden Antikörper erfolgen.

Durch das erfindungsgemäße Verfahren zum Nachweis von E-Cadherin mit monoklonalen Antikörpern wird es ermöglicht, mittels einfacher Untersuchung festzustellen, ob ein eher gutartiger oder bösartiger Tumor vorliegt, d.h. um das Risiko der Invasivität und Metastasierung einzuschätzen.

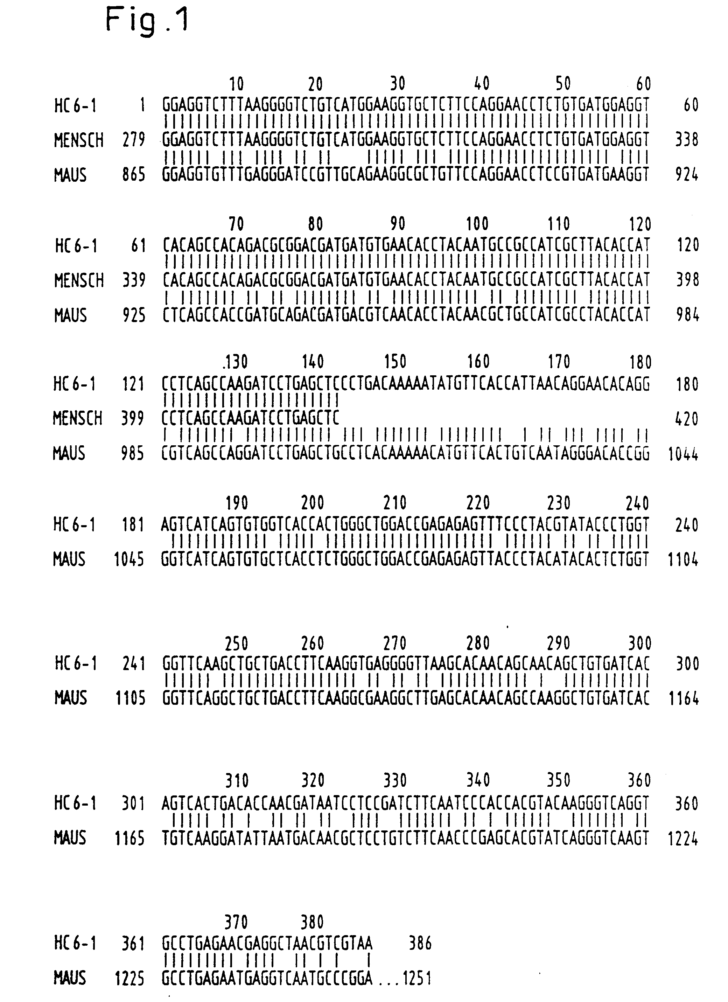

Weiterhin ist es auch möglich, die Expression von E-Cadherin auf mRNA-Ebene nachzuweisen. Der Nachweis von mRNA erfolgt dabei auf an sich bekannte Weise durch Hybridisierung mit einer Nukleinsäure-Sonde. Die Nukleinsäure-Sonde kann z.B. ein cDNA-Fragment oder ein synthetisches Oligonukleotid sein. Sie muß jedoch zur E-Cadherin mRNA komplementär sein und eine ausreichende Länge besitzen, so daß ein eindeutiger Nachweis von E-Cadherin mRNA erfolgen kann. Die Nukleinsäure-Sonde kann dabei die gesamte für E-Cadherin kodierende cDNA oder einen Teil daraus umfassen. Vorzugsweise enthält die Nukleinsäure-Sonde ein mindestens 14 Basen langes, zu E-Cadherin mRNA komplementäres Fragment. Vorzugsweise kann die in Fig. 1 dargestellte DNA-Sequenz des Klons HC6-1 oder eine mindestens 14 Nukleotide lange Teilsequenz dieser DNA-Sequenz verwendet werden. Die für das erfindungsgemäße Verfahren verwendete Nukleinsäure-Sonde sollte markiert sein, d.h. sie enthält radioaktive oder auf andere Weise markierte (z.B. biotinylierte) Nukleinsäurereste. Die Markierung selbst kann auf eine dem Fachmann bekannte Weise am Ende oder/und innerhalb der Nukleinsäure-Sonde angebracht werden (z.B. durch Kinasierung, Nicktranslation, Random-Priming etc.).

Zum Nachweis von E-Cadherin mRNA wird die RNA aus Tumorzellen isoliert. Dies kann auf an sich bekannte Weise geschehen, vorzugsweise jedoch durch Lysis der Zellen mit einem nichtionischen Detergens in Gegenwart von RNAsin und anschließender heißer Extraktion mit Phenol. Gegebenenfalls kann anschließend eine Anreicherung der poly-A⁺-RNA erfolgen. Das Vorhandensein von E-Cadherin mRNA wird durch Northern Blot Hybridisierung mit der oben beschriebenen Nukleinsäure-Sonde durchgeführt. Dabei kann die RNA vor der Hybridisierung elektrophoretisch aufgetrennt werden und anschließend auf eine Membran transferiert werden, so daß eine eindeutige Identifizierung anhand ihrer Länge möglich ist. Alternativ kann die Methode der RNAase-Protektion angewandt werden. Ist eine elektrophoretische Auftrennung nicht erforderlich, so kann die E-Cadherin mRNA-Expression auch durch Dot-Blot-Hybridisierung nachgewiesen werden. Eine weitere Möglichkeit besteht darin, Gewebeschnitte mit der Methode der "in situ"-Hybridisierung auf E-Cadherin-Transkripte zu untersuchen.

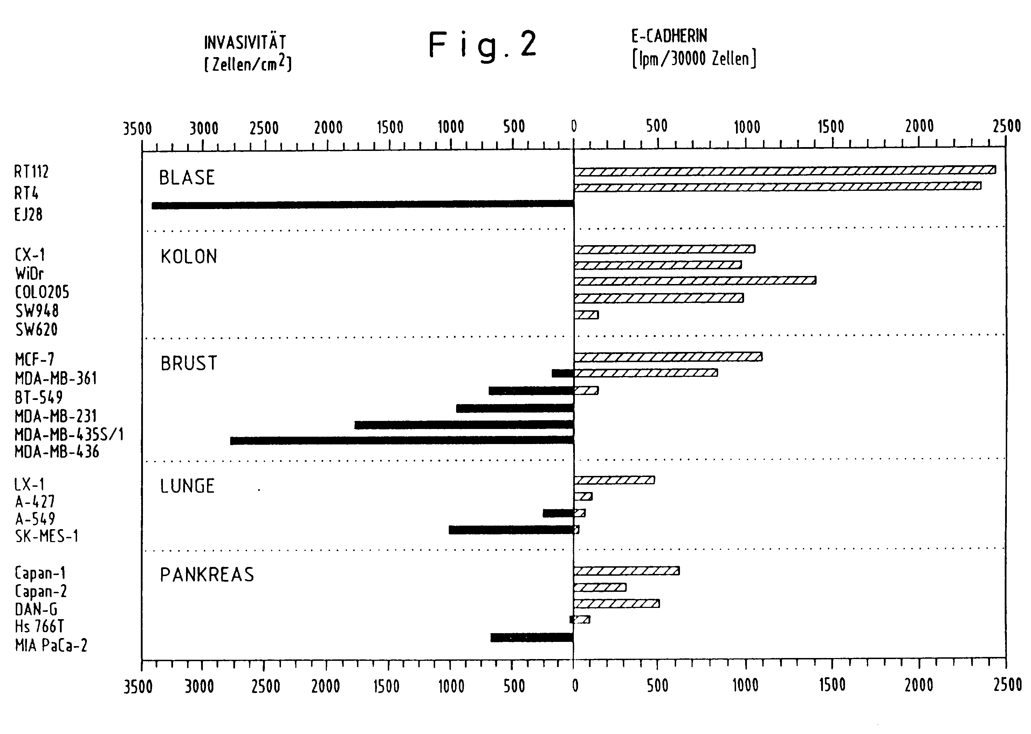

Die folgenden Beispiele erläutern in Verbindung mit den Figuren 1 und 2 die Erfindung weiter.
- Fig. 1: zeigt die Nukleotidsequenz des E-Cadherin cDNA-Klons HC6-1.
- Fig. 2: zeigt die Invasivität von Tumorzellinien in Korrelation zur E-Cadherin-Expression.

### Beispiel 1

### Herstellung von Antikörpern gegen E-Cadherin

1.1 Reinigung des 80 kd Trypsin-Fragments von menschlichem E-Cadherin
   Zur Herstellung des 80 kd Trypsin-Fragments von menschlichem E-Cadherin wurden A-431 Zellen (ATCC CRL 1555, menschliche Vulva-Karzinomzellen) mit einem Gummischaber von der Kulturschale abgelöst. Die Kultivierung von A-431 Zellen erfolgte wie in Beispiel 2.1 beschrieben. Das Zellgewebe wurde homogenisiert, abzentrifugiert, resuspendiert und mit Trypsin in Gegenwart von Ca²⁺ behandelt (Behrens et al., J. Cell. Biol. 108 (1989), 2435-2447). Das solubilisierte Material wurde auf einer Affinitätssäule chromatographiert, die durch Kopplung von 40 mg der IgG-Fraktion von Kaninchen-Anti Hund-Arc-1/Uvomorulin-Antiserum an 5 ml CNBr-Sepharose hergestellt wurde. Das 80 kd Trypsin-Fragment wurde spezifisch mit 5 mmol/l EDTA, 500 mmol/l NaCl, 50 mmol/l Tris-HCl, pH 8,5 eluiert und durch Acetonfällung bei -70°C konzentriert. Aus 1 g Naßzellen konnten gewöhnlich 1,5 µg des 80 kd Trypsin-Fragments von menschlichen E-Cadherin isoliert werden.
1.2 Herstellung monoklonaler Antikörper
   Zur Herstellung monoklonaler Antikörper wurden BALB/c-Mäuse mit dem Antigen (3 µg pro Tier und Immunisierung) immunisiert unter Verwendung des ABM-Adjuvans-Systems (Zymed Laboratories Inc., South San Francisco, CA). Milzzellen von immunisierten Mäusen wurden auf bekannte Weise mit P3-X63-Ag 8.653 Maus-Myelomzellen fusioniert (Kearney et al., J. Immunol. 123 (1983), 1548-1550). Die resultierenden Hybridom-zellinien wurden auf Gewebekulturplatten kultiviert.

Die Hybridom-Überstände wurden in einem Enzym-gekoppelten Immuntest unter Verwendung von Mikrotiterplatten mit 96 Löchern (NUNC, Dänemark) untersucht, die mit 75 ng pro Loch des Trypsin-Fragments von menschlichem E-Cadherin beschichtet waren. Antikörper mit Bindungs-Affinität an das Festphasen-Antigen wurden unter Verwendung von Peroxidase-gekoppelten Ziegen-Anti-Maus-Antikörpern gefunden, die spezifisch für IgG waren (Jackson Immuno Research Laboratories, West Grove, PA). Positive Hybridom-Überstände wurden durch Immunfluoreszenz auf menschlichen A-431-Karzinomzellen, Gefrierschnitten von menschlichem Dünndarm und durch Western-Blot-Analyse von A-431 Zellextrakten getestet. Zur Immunfluoreszenz von A-431-Zellen wurden die Kulturen mit 4 % Formaldehyd in PBS fixiert, mit 0,1 % Triton X-100 permeabilisiert und mit den Hybridom-Überständen und einem geeigneten zweiten, Fluorescein- oder Rhodamin-gekoppelten Antikörper inkubiert (Behrens et al., (1989), supra). Zur Immunfluoreszenz von Gefrierschnitten von menschlichem Dünndarm (8 µm) wurde das Gewebe mit Ethanol bei -20°C fixiert und wie beschrieben mit den Antikörpern inkubiert (Behrens et al., (1989), supra). Mit Western Blots wurden die verschiedenen Hybridom-Überstände auf Reaktion sowohl mit dem 80 kd Trypsin-Fragment von E-Cadherin und mit der 120 kd reifen Form des Moleküls getestet. Zur Sichtbarmachung der 120 kd Bande wurden frisch abgeschabte A-431-Zellen 5 Minuten lang bei 96°C mit 2 % SDS, 5 % 2-Mercaptoethanol in L-CAM-Testpuffer (Cunningham et al., Proc. Natl. Acad. Sci. USA 81 (1984), 5787-5791) lysiert. Anschließend erfolgte eine Zentrifugation bei 100.000 g (2 Stunden bei 12°C), SDS-Gelelektrophorese und Western Blot Analyse (siehe Behrens et al., Supra). Die Protein-A-Bindung der Antikörper wurde durch Adsorption der Hybridom-Überstände an fixierte Staphylococcus aureus Zellen in Gegenwart von 1 % Triton X-100 getestet, gefolgt von einer Analyse der SDS-Extrakte durch SDS-Polyacrylamid-Gelelektrophorese unter reduzierenden Bedingungen. Die Antikörper-Isotypen wurden unter Verwendung eines Maus-monoklonalen Isotyp-Kits (Serotec, Oxford, England) bestimmt.

Von 1200 getesteten Hybridom-Überständen enthielten etwa 30 IgG-Antikörper, die mit dem 80 kd-Fragment von E-Cadherin in einem Enzym-gekoppelten Immuntest reagierten. Davon waren 13 auch bei der Immunfluoreszenz, sowohl bei A-431 menschlichen Karzinomzellen (d.h., daß die Zell-Zell-Kontaktstellen gefärbt wurden) als auch an Gefrierschnitten des menschlichen Dünndarms (d.h., daß die lateralen Ränder der Epithelzellen gefärbt wurden) positiv. Diese Antikörper zeigten eine Immunreaktion mit dem 120 kd reifen E-Cadherin-Protein und mit dem 80 kd Trypsin-Fragment auf Western Blots. Weiterhin zeigten sie Bindung an Protein A. Die weiteren Experimente wurden mit 3 dieser Antikörper 6F9, 15C12 und 15G7 durchgeführt, die von den entsprechenden Hybridom-Zellinien sekretiert werden und hochspezifisch mit den 120 und 80 kd E-Cadherin-Banden bei Western Blots reagieren. Der Antikörper 6F9 ist von Bissendorf Chemikalien, Hannover und Eurodiagnosticfs kommerziell erhältlich. Die entsprechende Hybridomzellinie ist bei der GBF Braunschweig unter der Nummer ACC 1006 hinterlegt. Alle drei monoklonalen Antikörper 6F9, 15C12 und 15G7 waren in der Lage, spezifisch das Epithel von menschlichem Dünndarm anzufärben. Die Färbung war auf die lateralen Ränder der epithelialen Zellen beschränkt und eine Intensivierung der Färbung war im Bereich des Verbindungskomplexes zu sehen.

### Beispiel 2

Immunologischer Nachweis der E-Cadherin-Expression in verschiedenen menschlichen Tumor-Zellinien.
2.1 Zellinien
   Die verschiedenen getesteten, differenzierten und entdifferenzierten menschlichen Karzinom-Zellinien sind entweder bei der Tumorbank des Deutschen Krebsforschungszentrums (DKFZ) oder bei der American Type Culture Collection (ATCC) erhältlich. Die Literatur stellen, in denen die verschiedenen getesteten Zellinien beschrieben werden, sind aus Tabelle 1 ersichtlich. Die Zellinien wurden in DMEM mit 10 % foetalem Kalbserum unter Standard-Zellkultur-Bedingungen kultiviert.
2.2 immunologische Analyse der E-Cadherin-Expression
   Zur Immunfluoreszenz von E-Cadherin in Gewebekulturzellen wurden mit 100.000 g zentrifugierte Hybridomüberstände oder Protein A gereinigte Antikörper (mit 50 mmol/l Ethanolamin, pH 11 von Protein-A-Sepharose eluiert) verwendet. Die Immunfluoreszenz der Cytokeratine wurde nach Sun und Green, Cell 14, 469-476 1978) unter Verwendung von Breitspektrum-Antikörper (Jackson Immunoresearch Laboratories und Cammon Laborservice, Wiesbaden) durchgeführt. Für Western Blot Experimente wurden 50 µg des Gesamt-Zellproteins auf eine Gelspur geladen und die Nitrozellulose-Blots wurden mit den entsprechenden Hybridom-Überständen und einem geeigneten zweiten Antikörper inkubiert. Die quantitative Antikörper-Bindung an fixierte Gewebekultur-Zellen wurde im wesentlichen wie bei Behrens et al. (1989) supra beschrieben durchgeführt, abgesehen davon, daß ein Radiojodierter zweiter Antikörper verwendet wurde. Menschliche Karzinomzellen wurden über Nacht in flexiblen Mikrotiterplatten mit 96 Löchern (5x10⁴ Zellen pro Loch) kultiviert, mit Formaldehyd fixiert und mit Triton X-100 permeabilisiert. Nach Inkubation mit den entsprechenden Hybridom-Überständen (diese waren eine Stunde lang bei Raumtemperatur über fixierten MRC-5 menschlichen Lungenfibroblasten präabsorbiert) wurden die Zellen gewaschen und mit einen Ziegen-Anti-Maus-IgG (Jackson), markiert mit ¹²⁵I, unter Verwendung der Lactoperoxidase-Methode (2x10⁷ Ipm/µg, 5x10⁵ Ipm pro Loch) inkubiert. Die Löcher wurden dann ausgeschnitten und die Radioaktivität aus dreifach durchgeführten Experimenten in einem γ-Zähler bestimmt.

Es wurde gefunden, daß die Zellen aus differenzierten Tumoren im allgemeinen eine epitheloide Morphologie in Gewebekultur aufwiesen, während die aus entdifferenzierten Tumoren erhaltenen Zellen eine fibroblastoide Gestalt aufwiesen (siehe Tabelle 1).

Durch Immunofluoreszenz von Cytokeratinen unter Verwendung eines Breitspektrum-Antiserums wurde bestätigt, daß alle untersuchten Tumor-Zellinien epithelialen Ursprungs waren.

Durch immunologische Untersuchung mit den monoklonalen Antikörpern 6F9, 15G7 und 15C12 wurde gefunden, daß die differenzierten Karzinom-Zellinien im allgemeinen E-Cadherin exprimierten, während die entdifferenzierten Karzinom-Linien negativ für dieses Zell-Adhäsions-Molekül waren. Zum Beispiel wurde bei einem Immunfluoreszenz-Test mit dem Antikörper 6F9 wurde festgestellt, daß die differenzierte Blasen-Karzinom-Zellinie RT4 in Gebieten mit Zell-Zell-Kontakt E-Cadherin stark exprimierte, während die entdifferenzierte Blasen-Karzinom-Zellinie EJ28 keine Fluoreszenz und demnach auch keine Expression von E-Cadherin zeigte. Ebenso exprimierten die differenzierten Brust- (MCF-7 und MDA-MB-361), Lungen- (LX-1) und Pankreas- (DAN-G) Karzinom-Zellinien E-Cadherin, während die entsprechenden entdifferenzierten Brust- (MDA-MB-435S, MDA-MB-436 und MDA-MB-231), Lungen- (LXF289) und Pankreas- (MIA-PaCa2) Karzinom-Zellinien negativ waren. 4 differenzierte Colon-Karzinom-Zellinien (CX-1, WiDr COLO205 und HTC 116) waren ebenso im Immunfluoreszenz-Test positiv, während die entdifferenzierte Colon-Karzinom-Zellinien SW620 hauptsächlich aus spherischen Zellen ohne E-Cadherin-Expression und einer epitheloiden E-Cadherin-positiven Subpopulation bestand. Bei den intermediären Lungenkarzinom-Zellinien A-427 und A-549 exprimierten nur epitheloide Subpopulationen E-Cadherin.

Die relativen Färbintensitäten von E-Cadherin bei der Immunfluoreszenz korrespondieren mit der beim Westernblot gefundenen Menge des Moleküls. Zum Beispiel waren Extrakte der differenzierten Blasen- und Brust-Karzinom-Zellinien RT112, RT4 und MCF-7 positiv für das reife 120 kd Polypeptid, während die entdifferenzierten Blasen (EJ28) und die drei Brust MDA-MB-Karzinom-Zellinien negativ waren. Diese Unterschiede bei der Expression von E-Cadherin zwischen den epitheloiden und fibroplastoiden Karzinom-Zellinien wurden ebenso bei anderen getesteten Antikörpern, z.B. 15C12 und 15G7 gefunden.

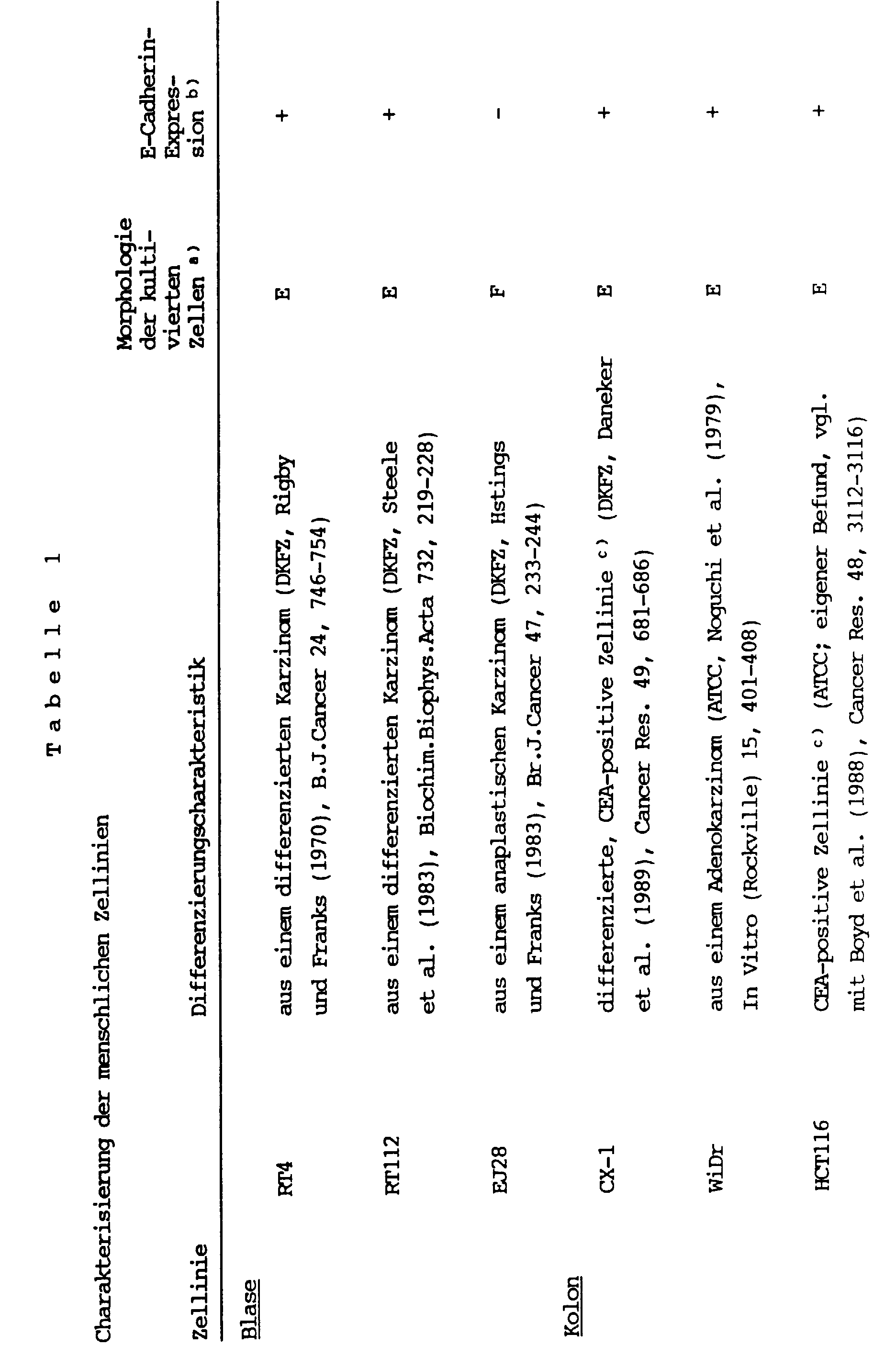

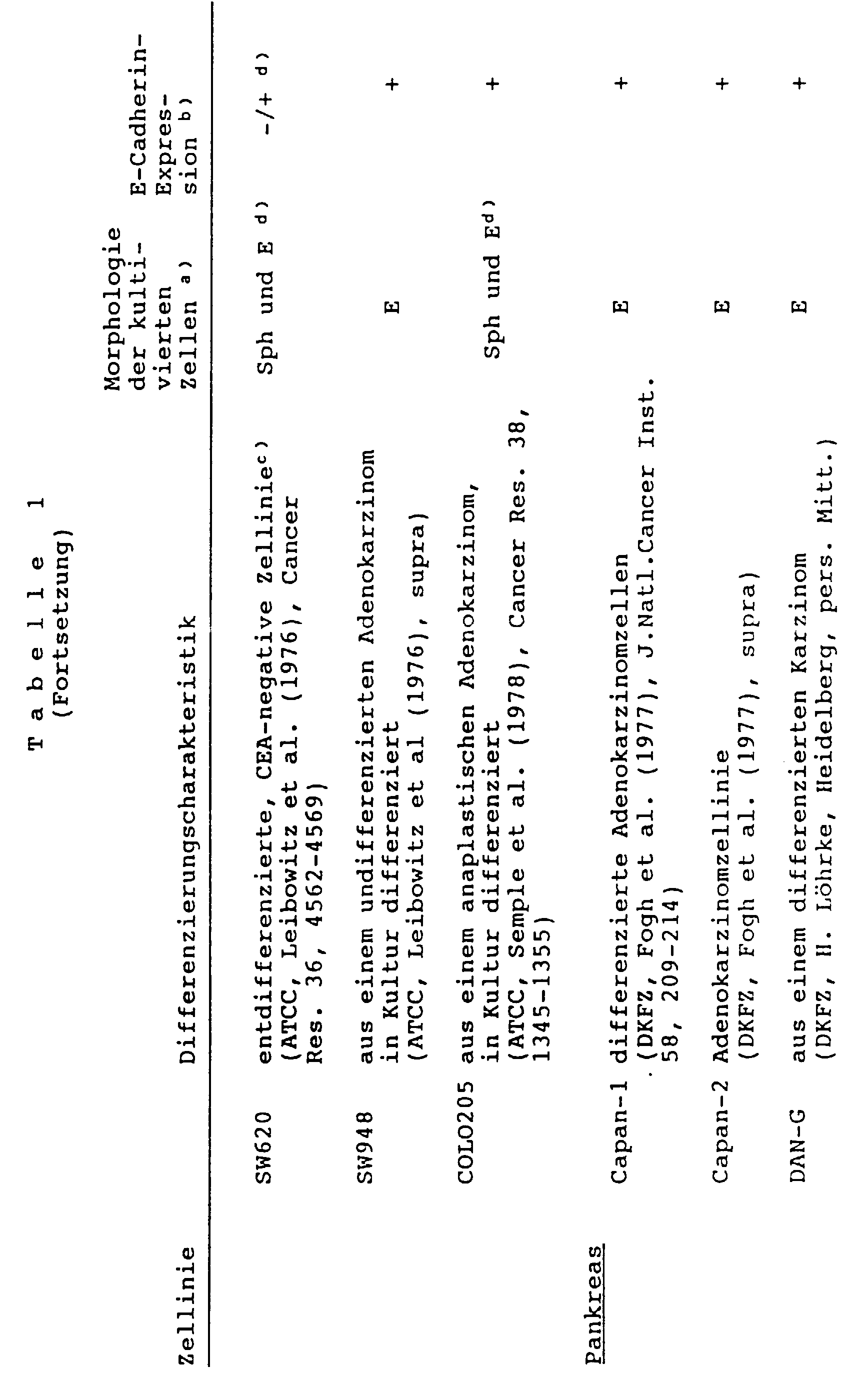

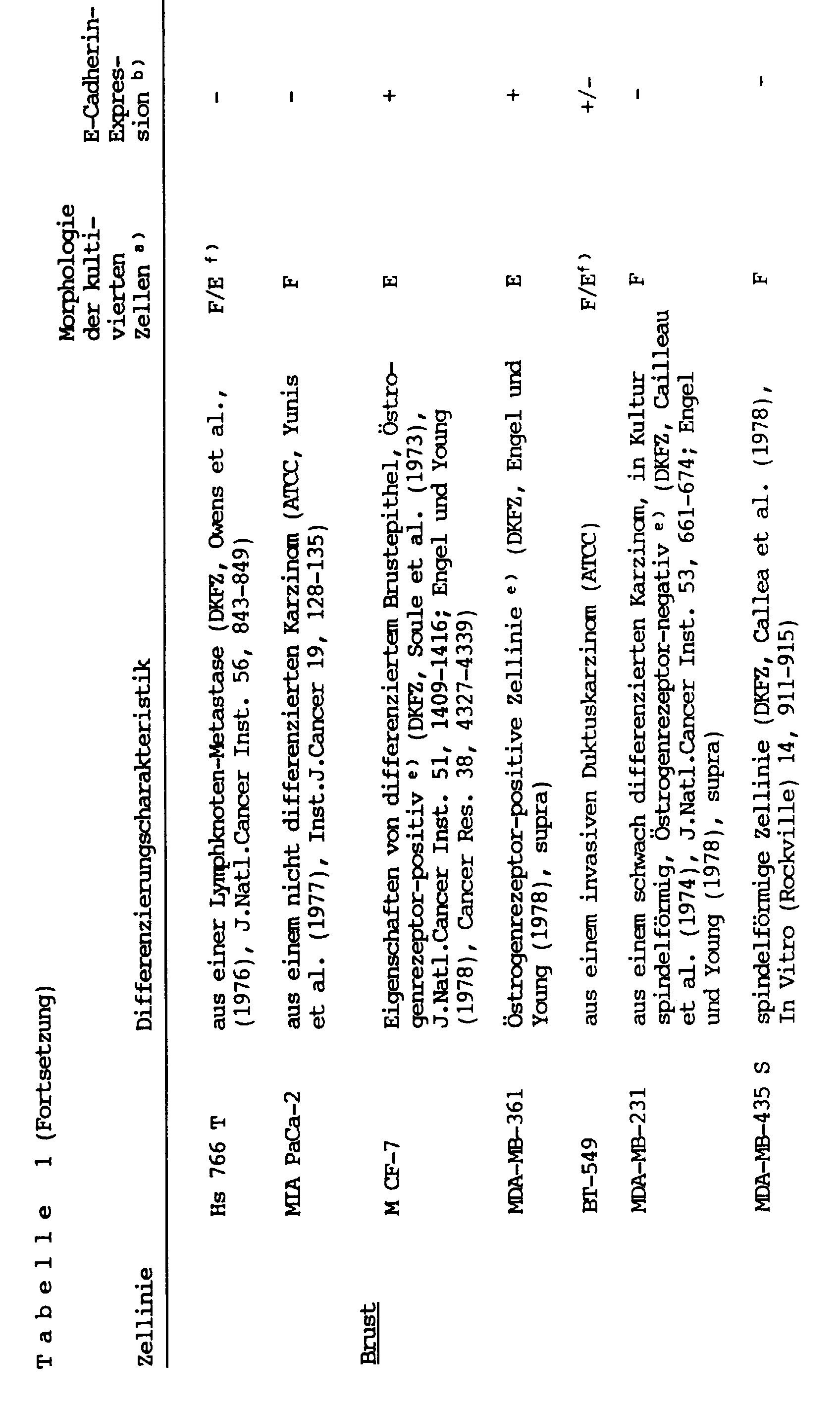

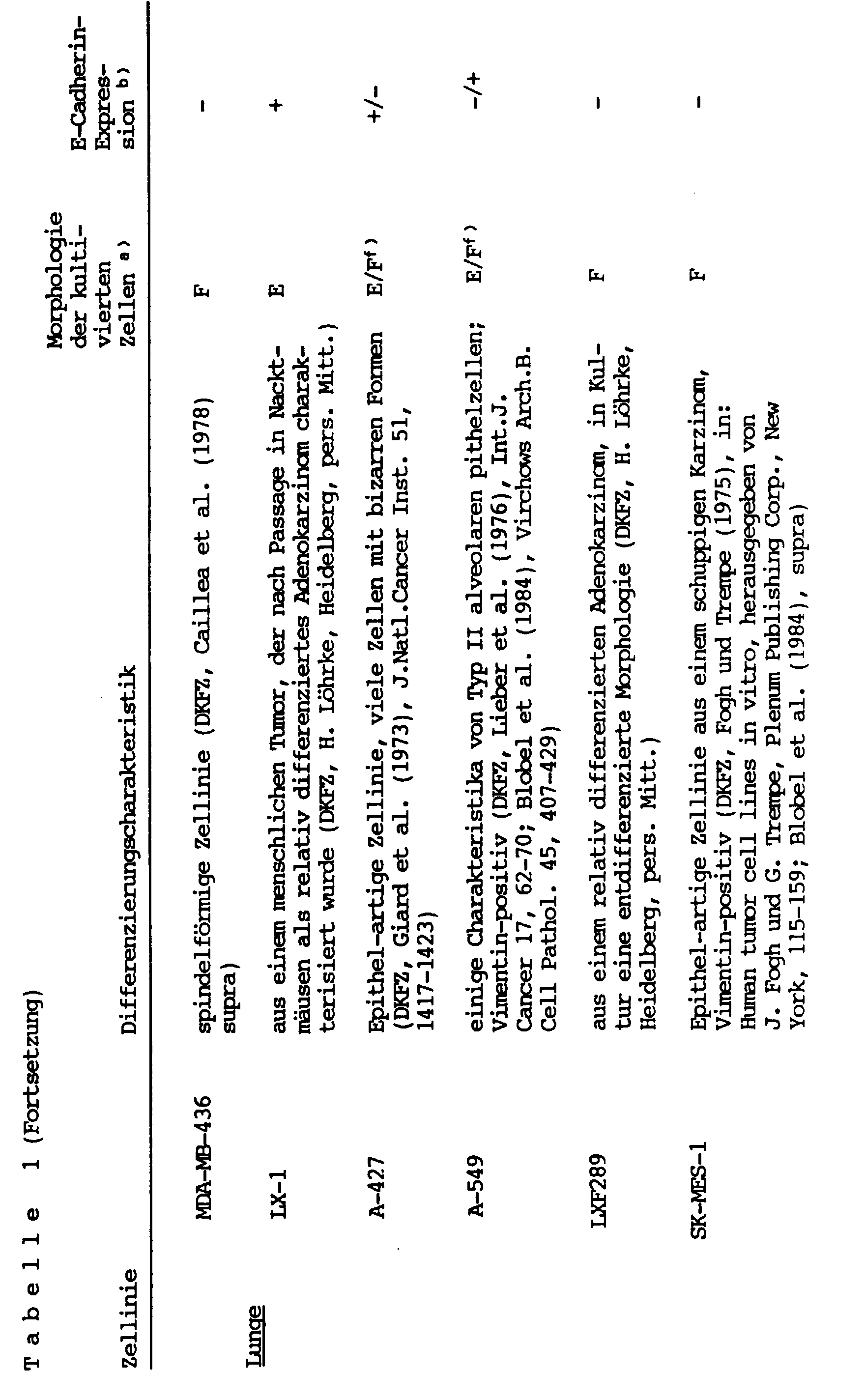

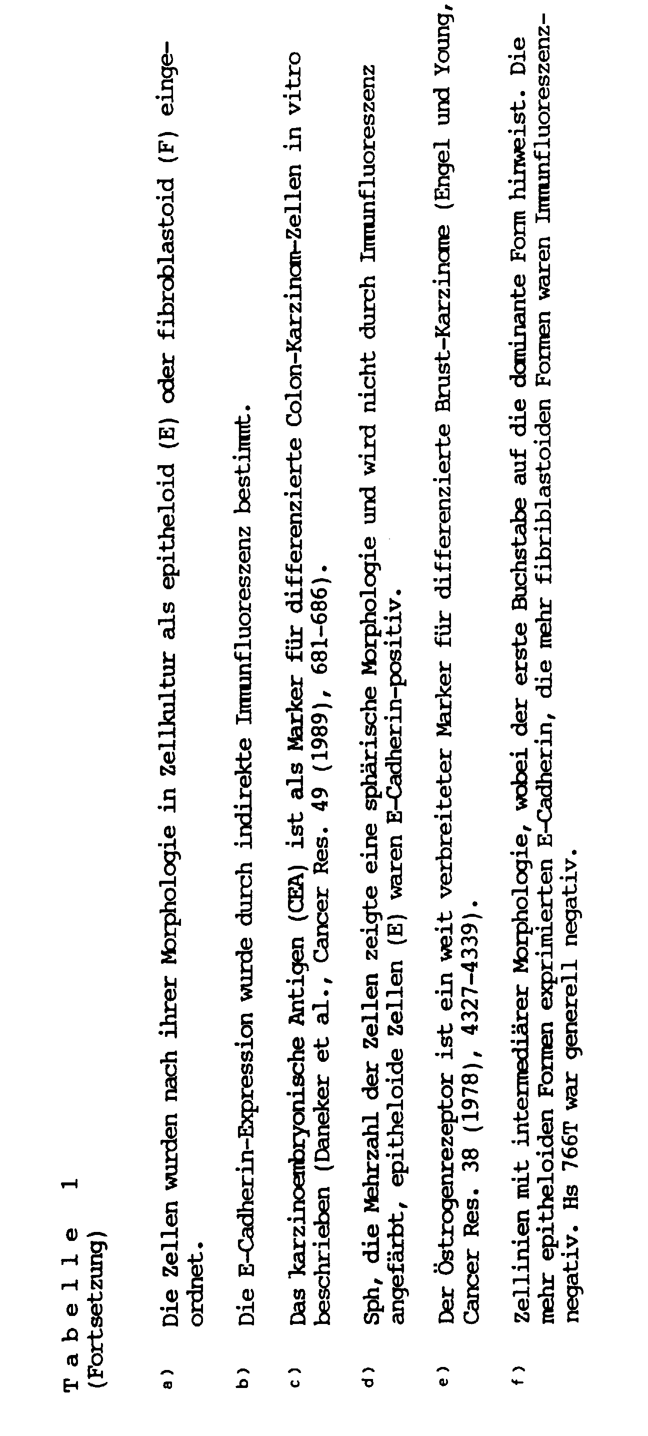

### Beispiel 3

### Nachweis von E-Cadherin auf mRNA-Ebene

3.1 Herstellung einer Nukleinsäure-Sonde
   Um eine geeignete cDNA-Probe zur Analyse des E-Cadherin-Gens und seiner Expression in den verschiedenen Karzinom-Zellinien zu erhalten, wurde eine menschliche Leber-cDNA-Bank (im kommerziell erhältlichen Plasmid-Expressions-Vektor pUEX1) untersucht. Aus 1,6x10⁵ Klonen wurde der positive Klon HC6-1 isoliert und die Insertion in M13-Phagen subkloniert und mittels der bideoxymethode unter Verwendung von T7-DNA-Polymerase (Pharmacia) in beiden Richtungen sequenziert. Die DNA-Sequenz der Insertion von HC6-1 ist in Fig. 1 dargestellt. Der partielle cDNA-Klon HC6-1 überlappt mit 142 bp einer anderen partiellen menschlichen cDNA (Mansouri et al., Differentiation 38 (1988), 67-71) und ist zur Maus cDNA homolog (Nagafuci et al., Nature 329 (1987), 341-343).
3.2 Isolierung von RNA aus Zellkulturen
   Kultivierte Zellen wurden mit PBS gewaschen, von den Platten mit einem Gummischaber entfernt und auf Eis in 1 % Nonidet P40, 140 mmol/l NaCl, 1,5 mmol/l MgCl₂, 100 Einheiten RNasin/ml, 10 mmol/l Tris-Cl, pH 8,0 lysiert. Nach Zentrifugation bei 12.000 Upm wurde der Überstand bei 65°C mit ungepuffertem Phenol mit 1 % SDS, 10 mmol/l EDTA extrahiert. Anschließend folgte eine Extraktion bei Raumtemperatur mit Tris-gepuffertem Phenol, Phenol/Chloroform, Chloroform. Dann erfolgte eine Präzipitierung der Nukleinsäuren bei -20°C mit Ethanol. Poly-A⁺-RNA wurde durch Affinitäts-Chromatographie der Gesamt-RNA an Oligo-dT-Zellulose unter Verwendung der Spun-Column-Methode (Pharmacia) erhalten. Die RNA wurde auf Glyoxalgelen elektrophoretisiert (Maniatis et al., Molecular Cloning (1982), Cold Spring Harbor Press, New York, USA). Dann erfolgte ein Blotten auf eine Hybond-N-Membran (Amersham). Die Hybridisierung der Blots mit der Nick-translatierten Nukleinsäure-Sonde wurde über Nacht bei 64°C in 5xSSC, 5x Denhardt's-Lösung, 10 % Dextransulfat (Pharmacia), 0,5 % SDS, 100 µg/ml erhitzte Lachssperma-DNA durchgeführt. Die Filter wurden bei Raumtemperatur mit 2xSSC (zweimal 10 Minuten) und anschließend mit 0,5xSSC, 0,1 % SDS bei 64°C (zweimal 15 Minuten) gewaschen, anschließend erfolgte eine Autoradiographie bei -70°C.

Bei Untersuchung der in Tabelle 1 dargestellten menschlichen Karzinom-Zellinien durch Northern Blot Analyse von Poly-A⁺-RNA unter Verwendung der HC6-1 cDNA-Probe wurden für die einzelnen Zellinien Unterschiede in der E-Cadherin-Expression gefunden, die denen der immunologischen Experimente entsprachen. Zum Beispiel exprimierten die differenzierten Karzinom-Zellinien RT112, RT4 (Blase), MCF-7 (Brust), LX-1 (Lunge), Capan-2, DAN-G (Pankreas), CX-1 und HCT116 (Colon) E-Cadherin-mRNA in hohem Ausmaß, während die korrespondierenden entdifferenzierten Karzinom-Zellinien EJ28 (Blase), die drei MDA-MB-Linien (Brust), SK-MES-1 und LXF289 (Lunge) keinerlei mRNA zeigten. Die Zellinien mit intermediären Phänotypen, z.B. A-427 (Lunge) und Hs 766T (Pankreas) exprimierten geringe Mengen an mRNA.

### Beispiel 4

### Test auf Invasivität von Tumor-Zellinien

4.1 Herstellung von Collagengelen
   Typ I Collagen (5 mg/ml, mit 1%iger Essigsäure aus Kalbshaut extrahiert, (Mauch et al., Exp. Cell Res. 163 (1986), 294-300) wurde gegen 0,05%ige Essigsäure dialysiert. 1/10 Volumen von 10fach konzentriertem DMEM (mit 22 mg/ml Natriumbicarbonat) wurde anschließend zugegeben. Dann wurde die Lösung neutralisiert. Aliquots von 1,2 ml pro Loch wurden zur Gelbildung in Sechsloch-Kulturschalen (Nunc) bei 37°C gegeben.
4.2 Test auf Invasivität
   Zur Durchführung der Tests auf Invasivität wurden 4x10⁵ Zellen (in 1,5 ml DMEM und 10 % foetalem Kalbserum) pro Loch auf die Collagenoberflächen ausplattiert. Nach dreitägiger Kultivierung auf den Collagengelen erfolgte eine Quantifizierung der invasiven Zellen im Lichtmikroskop (Behrens et al., (1989), supra). Die Expression von E-Cadherin wurde in einem indirekten Zellbindungstest unter Verwendung des monoklonalen Antikörpers 6F9 und 125 I markiertem Ziegen-Anti-Maus-IgG als zweitem Antikörper bestimmt.

Beim Test der menschlichen Karzinom-Zellinien auf Invasivität in vitro wurde gefunden, daß die Zellinien mit E-Cadherin-Expression im allgemeinen nicht in Collagen-Gele eindrangen, während die E-Cadherin negativen Zellinien invasiv waren (Abb. 2). Dies ist besonders deutlich bei den Blasen- und Brust-Karzinom-Zellinien. Bei den Brust- und Lungen-Karzinom-Zellinien ist eine stufenweise Veränderung vom E-Cadherinexprimierenden nicht invasiven Zustand zum nicht-exprimierenden invasiven Zustand klar erkennbar. Die differenzierten Colon- und Pankreas-Karzinom-Zellinien waren ebenfalls nicht invasiv. Die intermediäre Pankreas-Karzinom-zellinie Hs 766T zeigte nur eine geringe Invasivität.

Eine entdifferenzierte Karzinom-Zellinie (LXF289) ist in Abb. 2 nicht aufgeführt, da sie sich schlecht an die Mikrotiterschalen anlagerte und daher die Bindung nicht quantifiziert werden konnte. Diese Zellinie war in allen anderen Tests eindeutig E-Cadherin-negativ und invadierte das Collagen-Gel (322 Zellen pro cm²). Die entdifferenzierte menschliche Vulva-Karzinom-Zellinie A431, die zur Gewinnung von E-Cadherin verwendet wurde, war im Gelinvasionstest negativ.

## Patentansprüche

1. Verfahren zum Nachweis der Differenzierung oder/und Invasivität von Tumorzellen und Tumoren epithelialen Ursprungs, **dadurch gekennzeichnet**, daß man die Expression des oberflächenantigens E-Cadherin (L-CAM, Arc-1, Uvomorulin, cell-CAM 120/30) in Tumorzellen und Tumoren auf Proteinebene oder/und auf mRNA-Ebene untersucht.

2. Verfahren nach Anspruch 1, **dadurch gekennzeichnet,** daß man Gewebeschnitte oder/und Extrakte von Tumorzellen und Tumoren auf Anwesenheit von E-Cadherin oder eines Fragments davon durch Bindung eines monoklonalen Antikörpers gegen E-Cadherin untersucht.

3. Verfahren nach Anspruch 1, **dadurch gekennzeichnet**, daß man Gewebeschnitte oder/und Extrakte von Tumorzellen und Tumoren auf Anwesenheit von E-Cadherin mRNA durch Hybridisierung mit einer, der E-Cadherin mRNA komplementären Nukleinsäuresonde, untersucht.

4. Verfahren nach Anspruch 3, **dadurch gekennzeichnet**, daß man als Nukleinsäure-Sonde zum Nachweis der E-Cadherin mRNA die gesamte für E-Cadherin kodierende cDNA oder ein mindestens 14 Nukleotide langes Fragment davon verwendet.

5. Verfahren nach Anspruch 4, **dadurch gekennzeichnet**, daß man eine Nukleinsäure-Sonde aus der in Figur 1 dargestellten Nukleinsäuresequenz des E-Cadherin-Klons HC6-1 verwendet.

6. Verwendung einer zu E-Cadherin mRNA komplementären Nukleinsäure-Sonde zum Nachweis der Differenzierung oder/und Invasivität von Tumorzellen epithelischen Ursprungs.

7. Verwendung nach Anspruch 6, **dadurch gekennzeichnet**, daß die Nukleinsäure-Sonde die in Figur 1 dargestellte Nukleinsäuresequenz des E-Cadherin-Klons HC6-1 oder ein mindestens 14 Basen langes Fragment der E-Cadherin cDNA enthält.

## Claims

1. Method for the detection of the differentiation or/and invasiveness of tumour cells and tumours of epithelial origin,
**wherein**
the expression of the surface antigen E-cadherin (L-CAM, Arc-1, uvomorulin, cell-CAM 120/80) is examined in tumour cells and tumours at the protein level or/and at the mRNA level.

2. Method as claimed in claim 1,
**wherein**
tissue sections or/and extracts of tumour cells and tumours are examined for the presence of E-cadherin or a fragment thereof by binding of a monoclonal antibody against E-cadherin.

3. Method as claimed in claim 1,
**wherein**
tissue sections or/and extracts of tumour cells and tumours are examined for the presence of E-cadherin mRNA by hybridization with a nucleic acid probe which is complementary to E-cadherin mRNA.

4. Method as claimed in claim 3,
**wherein**
the entire cDNA coding for E-cadherin or a fragment thereof which is at least 14 nucleotides in length is used as the nucleic acid probe for the detection of E-cadherin mRNA.

5. Method as claimed in claim 4,
**wherein**
a nucleic acid probe is used from the nucleic acid sequence of the E-cadherin clone HC6-1 shown in Fig. 1.

6. Use of a nucleic acid probe which is complementary to E-cadherin mRNA for the detection of the differentiation or/and invasiveness of tumour cells of epithelial origin.

7. Use as claimed in claim 6,
**wherein**
the nucleic acid probe contains the nucleic acid sequence of the E-cadherin clone HC6-1 shown in Figure 1 or a fragment of the E-cadherin cDNA which is at least 14 bases long.

## Revendications

1. Méthode pour déterminer la différentiation ou/et l'invasivité de cellules tumorales et tumeurs d'origine épithéliale, **caractérisée en ce que** l'expression de l'antigène de surface E-cadherin (L-CAM, Arc-1, uvomorulin, cell-CAM 120/80) est examinée dans des cellules tumorales et des tumeurs au niveau de protéique ou/et au niveau d'A.R.N.-m.

2. Méthode selon la revendication 1,
**caractérisée en ce que** des sections de tissus ou/et des extraits de cellules tumorales et de tumeurs sont examinés en vue de détecter la présence éventuelle de E-cadherin ou d'un fragment de ce dernier en liant un anticorps monoclonal dirigé contre E-cadherin.

3. Méthode selon la revendication 1,
**caractérisée en ce que** des sections de tissus ou/et des extraits de cellules tumorales et de tumeurs sont examinés en vue de détecter la présence éventuelle de E-cadherin A.R.N.-m. par hybridation avec une sonde d'acide nucléique complémentaire de E-cadherin A.R.N.-m.

4. Méthode selon la revendication 3,
**caractérisée en ce que** tout l'A.D.N.-c. codant E-cadherin ou un fragment de celui-ci, d'une longueur minimale de 14 nucléotides, est employé comme sonde d'acide nucléique en vue de détecter E-cadherin A.R.N.-m.

5. Méthode selon la revendication 4,
**caractérisée en ce que** l'on utilise une sonde d'acide nucléique de la séquence d'acide nucléique du clone de E-cadherin HC6-1, représenté à la figure 1.

6. Emploi d'une sonde d'acide nucléique complémentaire de E-cadherin A.R.N.-m. pour détecter la différentiation ou/et l'invasivité de cellules tumorales d'origine épithéliale.

7. Emploi selon la revendication 6, **caractérisé en ce que** la sonde d'acide nucléique contient la séquence d'acide nucléique du clone de E-cadherin HC6-1, représentée à la figure 1, ou un fragment de E-cadherin A.D.N.-c., d'une longueur minimale de 14 bases.
